Europäisches Patentamt

European Patent Office

Office européen des brevets

(19)

(11) **EP 1 133 472 B1**

(12) **EUROPÄISCHE PATENTSCHRIFT**

(45) Veröffentlichungstag und Bekanntmachung des Hinweises auf die Patenterteilung:
**23.02.2005 Patentblatt 2005/08**

(21) Anmeldenummer: **99961004.1**

(22) Anmeldetag: **25.11.1999**

(51) Int Cl.$^7$: **C07C 319/24**, C07C 321/14

(86) Internationale Anmeldenummer:
**PCT/EP1999/009145**

(87) Internationale Veröffentlichungsnummer:
**WO 2000/031029 (02.06.2000 Gazette 2000/22)**

(54) **VERFAHREN ZUR HERSTELLUNG VON ORGANISCHEN DISULFIDEN**

METHOD OF PRODUCING ORGANIC DISULFIDES

PROCEDE POUR PREPARER DES BISULFURES ORGANIQUES

(84) Benannte Vertragsstaaten:
**AT BE CH CY DE DK ES FI FR GB GR IE IT LI LU MC NL PT SE**

(30) Priorität: **25.11.1998 DE 19854427**

(43) Veröffentlichungstag der Anmeldung:
**19.09.2001 Patentblatt 2001/38**

(73) Patentinhaber: **BASF AKTIENGESELLSCHAFT**
**67056 Ludwigshafen (DE)**

(72) Erfinder:
  • **HESSE, Werner**
    **D-67283 Obrigheim (DE)**
  • **STERZEL, Hans-Josef**
    **D-67125 Dannstadt-Schauernheim (DE)**

  • **TRAGUT, Christian**
    **D-67157 Wachenheim (DE)**

(74) Vertreter: **Isenbruck, Günter, Dr. et al**
    **Isenbruck, Bösl, Hörschler, Wichmann, Huhn,**
    **Patentanwälte**
    **Theodor-Heuss-Anlage 12**
    **68165 Mannheim (DE)**

(56) Entgegenhaltungen:
    **EP-A- 0 202 420        WO-A-98/34914**
    **GB-A- 987 358          US-A- 2 820 062**

Bemerkungen:
    Die Akte enthält technische Angaben, die nach dem Eingang der Anmeldung eingereicht wurden und die nicht in dieser Patentschrift enthalten sind.

**Beschreibung**

Die vorliegende Erfindung betrifft ein Verfahren zur Herstellung von organischen Disulfiden.

Aus dem Stand der Technik sind verschiedene Verfahren zur Herstellung von organischen Disulfiden bekannt.

In EP-A 0 171 092 wird ein Verfahren zur Herstellung von Dialkyldisulfiden beschrieben, in dem Alkohol, Schwefel und Schwefelwasserstoff direkt zu Dialkyldisulfiden und Wasser umgesetzt werden. Das Verfahren wird vorzugsweise an einem Katalysator auf Zeolithbasis durchgeführt, bei Temperaturen von 200 bis 400°C und in einem Druckbereich von Normaldruck bis 600 psig. Nachteilig bei diesem Verfahren ist, daß vielkomponentige Produktgemische erhalten werden, aus denen das Dialkyldisulfid nur in schlechten Ausbeuten (etwa 50 % der theoretischen Ausbeute) gewonnen werden kann.

In US 5,202,494 wird ein Verfahren beschrieben, bei dem Mercaptan mit Sauerstoff an einem MgO/Na$_2$O-dotierten Aluminiumoxidkatalysator zu Dialkyldisulfid und Wasser umgesetzt wird. Dabei entstehen Schwersieder, die bei der destillativen Aufarbeitung ausgeschleust werden müssen.

FR-B 1 358 398 offenbart ein Verfahren zur Herstellung von Dialkyldisulfiden aus Mercaptanen und elementarem Schwefel, in dem der Schwefel in Form einer Lösung in einem organischen Lösungsmittel, insbesondere in einem organischen Dialkyldisulfid eingesetzt wird. Als Katalysatoren dienen Amine. Nachteilig bei diesem Verfahren ist, daß von sauberen, und damit teuren, Mercaptanen ausgegangen werden muß.

Aufgabe der vorliegenden Erfindung ist es daher, ein Verfahren zur Herstellung von organischen Disulfiden bereitzustellen, das von preiswerten und gut verfügbaren Rohstoffen ausgeht und das gewünschte Dialkyldisulfid in guten Ausbeuten bereitstellt.

Die Aufgabe wird gelöst durch ein Verfahren zur kontinuierlichen Herstellung von organischen Disulfiden, umfassend die folgenden Schritte:

(a) Umsetzung von Alkanolen mit Schwefelwasserstoff an einem geeigneten Katalysator zu einem "Roh-Mercaptan-Strom" enthaltend Mercaptan, Wasser, Schwefelwasserstoff sowie geringe Mengen weiterer Nebenprodukte wie organisches Sulfid und Ether,

(b) Umsetzung des "Roh-Mercaptan-Stroms" mit in einem organischen Disulfid gelöstem Schwefel unter Katalyse mit einem Amin, das flüssig oder fest ist und einen Siedepunkt oberhalb des Siedepunkts des eingesetzten Disulfids und eine Wasserlöslichkeit von weniger als 0,5 g/l aufweist, in einer Reaktionskolonne, wobei anfallende Leichtsieder in Schritt (a) zurückgeführt werden,

(c) Phasentrennung des erhaltenen Gemisches aus wäßriger Phase, die ausgeschleust wird und schwefelorganischer Phase,

(d) Aufreinigung der schwefelorganischen Phase, die gegebenenfalls Leichtsieder, das gewünschte organische Disulfid, Polysulfide, Amin und geringe Mengen weiterer Nebenprodukte enthält, durch Destillation, wobei das organische Disulfid entnommen wird, gegebenenfalls anfallende Leichtsieder in Schritt (a) zurückgeführt werden und anfallende Polysulfide und Amin in Schritt (b) zurückgeführt werden, unter Zusatz von Schwefel und gegebenenfalls Amin,

wobei die Phasentrennung und das Ausschleusen der wäßrigen Phase in Schritt (c) im Anschluß an Schritt (a) oder Schritt (b) erfolgen kann.

Im Sinne der vorliegenden Erfindung ist unter einem "Roh-Mercaptan-Strom" ein nicht durch Extraktion oder Destillation gereinigter Mercaptan-Strom aus der Synthese von Mercaptanen aus Alkoholen und Schwefelwasserstoff zu verstehen. Der "Roh-Mercaptan-Strom" kann noch nicht-abreagierten Schwefelwasserstoff, Wasser und als Nebenkomponenten Dialkylsulfid, geringe Mengen Alkohol und Dialkylether enthalten.

Üblicherweise wird ein solcher "Roh-Mercaptan-Strom" durch eine aufwendige, mehrstufige Druckdestillation aufgereinigt, was hohe Kosten verursacht. Durch den Verzicht auf die Destillationsstufe wird eine Druckstufe vermieden, und das gesamte Verfahren kann drucklos durchgeführt werden. Dadurch wird ein Kostenvorteil erreicht, da keine aufwendigen Druckapparaturen benötigt werden. Gleichzeitig wird eine erhöhte Sicherheit gewährleistet.

Das eingesetzte "Roh-Mercaptan" wird durch Umsetzung von Alkohol und Schwefelwasserstoff an einem zur Mercaptan-Synthese geeigneten Katalysator hergestellt. Vorzugsweise wird die Umsetzung bei Temperaturen zwischen 350 und 450°C in der Gasphase durchgeführt. Als Katalysatoren können alle, dem Fachmann bekannten, zur Mercaptan-Synthese geeigneten Katalysatoren eingesetzt werden. Bevorzugt werden dotierte Aluminium-Oxide eingesetzt, wobei als Dotierungen 1. KOH (EP-A 0 564 706), 2. K$_2$CO$_3$ (EP-A 0 749 961), 3. B$_2$O$_3$, K$_3$WO$_4$ (React. Kinet. Catal. Lett. Bd. 36, 1, S. 159), 4. CaO (EP-A 0 564 706) verwendet werden. Besonders bevorzugt wird als Katalysator Kalium-Wolframat auf aktiviertem Al$_2$O$_3$ eingesetzt in Verfahren, wie sie in US 3,935,276 und Hillis O. Folkins und Elmer L. Miller, I&EC Process Design and Development, Vol. 1, Nr. 4, Oktober 1962, beschrieben werden. Aus Gründen eines verminderten Emissionsrisikos arbeitet man in dem erfindungsgemäßen Verfahren dabei üblicherweise bei 1 bis 3 bar absolut, bevorzugt annähernd drucklos, anstelle der sonst üblichen 10 bis 15 bar.

Zur Erzeugung des "Roh-Mercaptan-Stroms" wird vorzugsweise ein einfacher Rohrreaktor mit gasförmigem Schwefelwasserstoff und Alkohol gespeist und an einem festen Katalysator, bevorzugt Kalium-Wolframat auf aktiviertem Al$_2$O$_3$, umgesetzt. Dieser Gasstrom wird mit in einem organischen Disulfid gelöstem Schwefel unter Einsatz eines Amins als Katalysator zu organischen Disulfiden oxidiert.

Vorzugsweise handelt es sich bei dem als Lösungsmittel eingesetzten organischen Disulfid und dem herzustellenden organischen Disulfid um dieselbe Verbindung. Dadurch wird die Abtrennung von zusätzlichem Lösungsmittel eingespart.

Über die Reaktion bestehen folgende Vorstellungen:

Beim Lösen von Schwefel in organischen Disulfiden bilden sich organische Polysulfide. Unter organischen Polysulfiden sind erfindungsgemäß Sulfide der allgemeinen Formel R(-S)$_n$-R zu verstehen, worin die Reste R abhängig von dem eingesetzten organischen Disulfid und dem eingesetzten Mercaptan sind - bei Anwesenheit des Mercaptans in der eigentlichen Reaktion - d.h. R muß nicht gleichartig sein, bevorzugt ist es dies aber, um zusätzliche Aufarbeitungsschritte zu vermeiden (Endprodukt und Lösungsmittel bevorzugt gleich). n ist im allgemeinen eine ganze Zahl von 3 bis 12, bevorzugt von 3 bis 9. In konzentrierteren Lösungen ist zusätzlich auch physikalisch gelöster S$_8$-Schwefel enthalten. Der gelöste Schwefel und die organischen Polysulfide (nachstehend gemeinsam mit "S" symbolisiert) reagieren bei Temperaturen zwischen Raumtemperatur und dem Siedepunkt des herzustellenden organischen Disulfides in Anwesenheit von Mercaptanen nach folgender Gleichung zu organischen Disulfiden ab:

$$2\ RSH + "S" \rightarrow R\text{-}S\text{-}S\text{-}R + H_2S$$

Dabei reagieren der gelöste Schwefel und die höheren Polysulfide am schnellsten ab, während niedere Polysulfide bis hin zum Trisulfid (n=3) nur langsam abreagieren. Aufgrund des im "Roh-Mercaptan-Strom" immer noch enthaltenen Schwefelwasserstoffs, wird z. B. ein Gleichgewicht gemäß der folgenden Gleichung zwischen organischen Disulfiden und organischen Trisulfiden angenommen:

$$2\ R\text{-}SH + R\text{-}S\text{-}S\text{-}S\text{-}R \rightleftarrows 2\ R\text{-}S\text{-}S\text{-}R + H_2S$$

Gebildetes organisches Trisulfid reagiert demgemäß nicht vollständig zu organischem Disulfid ab. Als Endprodukt wird also kein reines polysulfidfreies organisches Disulfid erhalten; sondern immer ein Gemisch aus organischen Disulfiden und organischen Trisulfiden, das weiter aufgereinigt wird. Des weiteren ist im Produktgemisch noch das als Katalysator eingesetzte Amin enthalten.

Als Amine können in dem erfindungsgemäßen Verfahren primäre, sekundäre und tertiäre aliphatische oder aromatische Amine eingesetzt werden. Vorzugsweise werden primäre, sekundäre oder tertiäre aliphatische Amine eingesetzt. Die Amine sind flüssig oder fest mit einem Siedepunkt oberhalb des Siedepunktes der eingesetzten organischen Disulfide und einer Wasserlöslichkeit von weniger als 0,5 g/l. Ganz besonders bevorzugt haben die eingesetzten Amine einen Siedepunkt oberhalb von 140°C und eine Wasserlöslichkeit von kleiner als 0,1 g/l. Insbesondere handelt es sich bei den ganz besonders bevorzugten Aminen um primäre, sekundäre oder tertiäre Amine mit 6 bis 60 Kohlenstoffatomen. Beispielsweise sind Tridecylamin, Fettamine wie N,N-Dimethyl-C$_{12}$/C$_{14}$-amin, Dicyclohexylamin geeignet. Das Amin wird im allgemeinen in einer Menge von 0,1 bis 10 Gew.-%, bevorzugt von 0,5 bis 5 Gew.-%, besonders bevorzugt von 1 bis 3 Gew.-%, bezogen auf den eingesetzten Schwefel, eingesetzt.

Das erfindungsgemäße Verfahren eignet sich zur Herstellung von organischen Disulfiden aus beliebigen Mercaptanen. Im allgemeinen werden Mercaptane mit aliphatischen, cycloaliphatischen, Aryl-, Arylalkyl- oder Aralkylresten eingesetzt. Die aliphatischen Reste können linear oder verzweigt, mit funktionellen Gruppen wie Hydroxy-, Halogen-, Thio-, Thioether-, Sulfonyl-, Sulfoxyl-, Sulphenyl-, Amino-, Imino-, Nitro oder Nitrosogruppen substituiert oder unsubstituiert, gesättigt oder ein- oder mehrfach ungesättigt sein. Die cycloaliphatischen Reste können Doppelbindungen und/oder Heteroatome, insbesondere S oder N enthalten. Beispielsweise können Methyl-, Ethyl-, n-Propyl-, Isopropyl-, n-Butyl-, Isobutyl-, Amyl-, Hexyl- oder Benzylmercaptan eingesetzt werden. So erhält man Dimethyl-, Diethyl-, Di-n-propyl-, Diisopropyl-, Di-n-butyl-, Diisobutyl-, Diamyl-, Dihexyl- oder Dibenzyldisulfide. Bevorzugt werden Mercaptane mit gesättigten aliphatischen Resten mit 1 bis 3 Kohlenstoffatomen wie Methyl-, Ethyl- oder n-Propylmercaptan eingesetzt. Besonders bevorzugt ist Methylmercaptan. Das besonders bevorzugt hergestellte organische Disulfid ist demnach Dimethyldisulfid.

Das erfindungsgemäße Verfahren wird als kontinuierliches Verfahren durchgeführt. Besonders bevorzugt ist das Verfahren in sich geschlossen, das heißt, die als Nebenprodukte entstehenden Wertprodukte können wieder eingesetzt werden.

Vorzugsweise wird die Herstellung des "Roh-Mercaptan-Stroms" in einem einfachen Rohrreaktor in der Gasphase durchgeführt und der entstandene Gasstrom wird anschließend in den unteren Teil einer Reaktionskolonne, beispielsweise einer Glockenbodenkolonne, eingeleitet. In Schritt (b) trifft dieser Gasstrom im Gegenstrom auf eine Lösung von Schwefel in organischem Disulfid, in der das als Katalysator benötigte Amin gelöst ist. Am Kopf der Reaktionskolonne anfallende Leichtsieder können in Schritt (a) zurückgeführt werden. Im Sumpf der Reaktionskolonne wird organisches Disulfid im Gemisch mit organischem Trisulfid und gegebenenfalls weiteren niederen Polysulfiden erhalten. Des weiteren enthält der Sumpf der Reaktionskolonne noch das Amin, sowie aus der Mercaptansynthese eingetragenes Wasser.

Die Sumpftemperatur in der Reaktionskolonne beträgt im allgemeinen 20 bis 120°C, bevorzugt 50 bis 100°C, besonders bevorzugt 90 bis 95°C. Die Kopftemperatur beträgt im allgemeinen -20 bis +30°C, bevorzugt 0 bis +20°C, besonders bevorzugt 0 bis +10°C. Schritt (b) wird im allgemeinen drucklos durchgeführt.

Die wäßrige Phase wird in Schritt (c) von der schwefelorganischen Phase getrennt, vorzugsweise in einem Phasenabscheider, und entsorgt. Diese Phasentrennung kann auch direkt nach der Mercaptansynthese (Schritt (a)) erfolgen. Wird die wäßrige Phase im Anschluß an Schritt (b) abgetrennt, sollte die Sumpftemperatur in der Reaktionskolonne vorzugsweise über 80°C, bevorzugt zwischen 90 und 95°C betragen, so daß die auszutragende wäßrige Phase nur mit geringen Mengen an leichtsiedenden Schwefelverbindungen wie Schwefelwasserstoff belastet ist. Die Temperatur sollte andererseits nicht so hoch liegen, daß das Wasser Großteile der Kolonne belegt und nicht mehr über den Sumpf ausgetragen werden kann.

Ein Vorteil dieses erfindungsgemäßen Verfahrens ist, daß im Reaktionsgemisch enthaltener Schwefelwasserstoff vom Kopf der Reaktionskolonne zur Mercaptan-Synthese zurückgeführt werden kann. Auch Nebenkomponenten wie organisches Sulfid, Alkohol und Ether werden auf diesem Wege zurückgeführt und im Rohrreaktor zu Mercaptan umgesetzt (Schritt (a)).

Die schwefelorganische Phase wird nach Abtrennung des Wassers aufgereinigt (Schritt (d)). Die Aufreinigung erfolgt durch Destillation, besonders bevorzugt durch Destillation bei vermindertem Druck. Durch den verminderten Druck können zu hohe Sumpftemperaturen vermieden werden, wodurch eine Zersetzung von organischen Polysulfiden und Schwefel zu beispielsweise $CS_2$ und Mercaptan verhindert wird.

Die Destillation wird in einer Destillationskolonne durchgeführt, die vorzugsweise mindestens zehn theoretische Böden enthält. Geringe Mengen bei der Destillation erhaltene Leichtsieder wie Mercaptan, organisches Sulfid oder $CS_2$, werden abgetrennt und können in Schritt (a) zurückgeführt werden. Das organische Disulfid wird entnommen, wobei die Entnahme vorzugsweise in einem Seitenabzug der Destillationskolonne durchgeführt wird. Die Sumpflösung oder ein Teil dieser Sumpflösung wird in Schritt (b) zurückgeführt, indem elementarer Schwefel, vorzugsweise in flüssiger Form, in dieser Mischung gelöst wird und eventuell der Katalysator Amin nachdosiert wird und diese Mischung wieder in den oberen Teil der Reaktionskolonne eingeleitet wird.

Das erfindungsgemäße Verfahren stellt ein geschlossenes System bereit, in dem kontinuierlich nach der Bruttogleichung :

$$2\ ROH + H_2S + S \rightarrow R\text{-}S\text{-}S\text{-}R + 2\ H_2O$$

mittels eines Vorreaktors und zweier Kolonnen organische Disulfide hergestellt werden können. Die in dem erfindungsgemäßen Verfahren hergestellten organischen Disulfide können insbesondere zur Herstellung von Sulfonsäuren verwendet werden. Sie eignen sich dabei insbesondere zur Herstellung von Alkylsulfonsäuren, bevorzugt durch Oxidation der organischen Disulfide mit $HNO_3$.

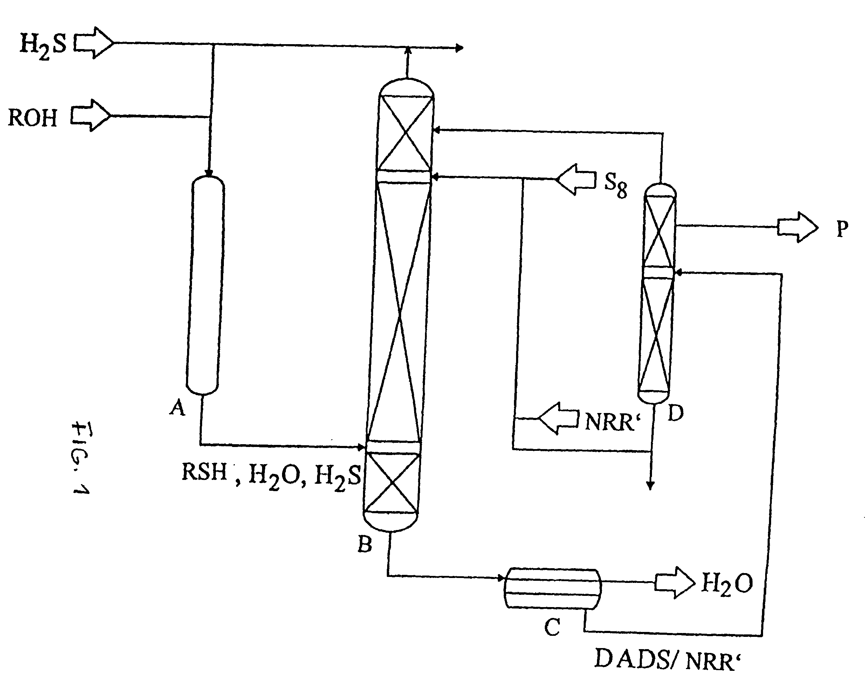

In der anliegenden Zeichnung zeigt Fig. 1 ein beispielhaftes Verfahrensschema zur Synthese der organischen Disulfide.

Darin bedeuten:

A        Rohrreaktor, in dem Schritt (a) durchgeführt wird;

B        Reaktionskolonne, in der Schritt (b) durchgeführt wird;

C        Phasenabscheider, in dem Schritt (c) durchgeführt wird;

D        Destillationskolonne, in der Schritt (d) durchgeführt wird;

NRR'     als Katalysator in Schritt (b) eingesetztes Amin;

P        gereinigtes Produkt (organisches Disulfid);

DADS     organisches Disulfid.

Die übrigen Abkürzungen ergeben sich aus dem vorstehenden Text.

Die folgenden Beispiele erläutern die Erfindung zusätzlich.

**Beispiele**

1. Beispiel in kontinuierlicher Versuchsanlage

In einer kontinuierlich arbeitenden Laborapparatur, die alle Komponenten des Fließschemas (Fig. 1) enthält, wurde die zweistufige Dimethyldisulfid-(DMDS)-Synthese durchgeführt.

Der MeSH-Rohrreaktor (600 mm lang, 25 mm Durchmesser) war mit einem selbst hergestellten Katalysator aus Aluminiumoxid mit 14 Gew.-% Kalium-Wolframat gefüllt. Bei einer Temperatur von 400°C (Reaktormitte) und einem Druck von 1,1 bar$_{abs}$ wurden 48 g/h (1,5 mol) gasförmiges Methanol, 34 g/h (1 mol) Schwefelwasserstoff und 40 g/h Rückgas aus der Reaktionskolonne (Zusammensetzung: 72 % $H_2S$, 12 % MeSH, 16 % Dimethylsulfid (DMS), geringe Mengen Methanol und Dimethylether) in den Rohrreaktor gespeist Die Gaszusammensetzung am Reaktorausgang war: 13,0 % $H_2S$, 50,8 % MeSH, 10,3 % DMS, 21,6 % $H_2O$, 2,4 % MeOH, 1,2 % Dimethylether (DME).

Dieses Gas tritt in den unteren Teil der Reaktionskolonne (Glockenbodenkolonne mit 20 Böden) ein und strömt im Gegenstrom zu der herablaufenden Schwefellösung (Sumpflösung aus Destillationskolonne, zu der 19 g/h Schwefel und 0,1 g/h Dicyclohexylamin zudosiert wird), wobei MeSH unter $H_2S$-Bildung reagiert. Das am Kolonnenkopf austretende Gas mit der obigen Zusammensetzung wird als Rückgas verdichtet und zum MeSH-Rohrreaktor zurückgefahren, wobei ein kleinerer Teil des Gases ausgeschleust wird. Die auf ca. 90°C geheizte Sumpflösung ist zweiphasig, wobei die obere Phase das in der MeSH-Synthese gebildete Wasser ist. Die Phasen werden in einen Phasenscheider gefahren, dort getrennt und die obere, wässrige Phase in einem Austragsbehälter gesammelt (26 g/h). Die untere, schwefelorganische Phase wird seitlich in eine Destillationskolonne gefahren, die bei Unterdruck (300 mbar) betrieben wurde. Das Produkt DMDS wurde im Seitenabzug isoliert (53 g/h) und hatte eine Reinheit von 99,6 % (0,1 % Dimethyltrisulfid, 0,3 % Leichtsieder wie Dimethylsulfid (DMS), MeSH, $CS_2$). Damit liegt die Ausbeute an DMDS bezogen auf den eingesetzten Schwefel bei 94,9 %. Die am Kopf der Destillationskolonne anfallenden Leichtsieder wurden in diesem Fall nicht wie in Fig.1 zum Kopf der Reaktionskolonne rückgeführt.

2. Vergleichsbeispiel

In einem thermostatisierten Glasrohr mit Fritte wurde eine Lösung aus 50 g Schwefel, 25 g DMDS, 1 g Triisobutylamin vorgelegt und auf 60°C thermostatisiert. Dann wurde von unten gasförmiges Methylmercaptan aus einer Stahlbombe in einer Menge von 24 g/h über die Fritte durch die Lösung geleitet. Die Zusammensetzungen von Gas- und Flüssigphasen wurden in Abhängigkeit der Zeit gaschromatographisch untersucht. Zu Beginn der Reaktion wurde ein Großteil des Methylmercaptans unter Bildung von Schwefelwasserstoff umgesetzt. Die Gasphase über der Flüssigkeit enthielt anfangs ca. 75 % $H_2S$. Der Gehalt an $H_2S$ sank dann ab, bis am Ende der Reaktion fast reines Methylmercaptan durch die Lösung trat. Die Reaktion war nach 13 h beendet. Es wurden 118 g Lösung erhalten, die folgende Zusammensetzung aufwies: 0,5 % $H_2S$, 5,8 % MeSH, 55,2 % DMDS, 32,9 % Dimethyltrisulfid, 4,2 % Dimethylpolysulfide, 0,9 % Amin.

3. Vergleichsbeispiel

In einem thermostatisierten Glasrohr mit Fritte wurde eine Lösung aus 50 g Schwefel, 25 g DMDS, 1 g Tridecylamin vorgelegt und auf 60°C thermostatisiert. Dann wurde von unten gasförmiges Methylmercaptan aus einer Stahlbombe in einer Menge von 24 g/h über die Fritte durch die Lösung geleitet. Die Zusammensetzungen von Gas- und Flüssigphasen wurden in Abhängigkeit der Zeit gaschromatographisch untersucht. Zu Beginn der Reaktion wurde ein Großteil des Methylmercaptans unter Bildung von Schwefelwasserstoff umgesetzt. Die Gasphase über der Flüssigkeit enthielt anfangs ca. 79 % $H_2S$. Der Gehalt an $H_2S$ sank dann ab, bis am Ende der Reaktion fast reines Methylmercaptan durch die Lösung trat. Die Reaktion war nach 12 h beendet. Es wurden 156 g Lösung erhalten, die folgende Zusammensetzung aufwies: 0,1 % $H_2S$, 7,7 % MeSH, 90,5 % DMDS, 0,8 % Dimethyltrisulfid, keine Dimethylpolysulfide, 0,8 % Amin. Die Ausbeute an DMDS bezogen auf den eingesetzten Schwefel liegt bei 83 %. Die Verluste kommen durch den gasförmigen Austrag an DMDS mit den Reaktionsgasen zustande.

## Patentansprüche

1. Verfahren zur kontinuierlichen Herstellung von organischen Disulfiden umfassend die folgenden Schritte

   (a) Umsetzung von Alkanolen mit Schwefelwasserstoff an einem geeigneten Katalysator zu einem "Roh-Mercaptan-Strom" enthaltend Mercaptan, Wasser, Schwefelwasserstoff sowie geringe Mengen weiterer Nebenprodukte wie organisches Sulfid und Ether,

   (b) Umsetzung des "Roh-Mercaptan-Stroms" mit in einem organischen Disulfid gelöstem Schwefel unter Katalyse mit einem Amin, das flüssig oder fest ist und einen Siedepunkt oberhalb des Siedepunkts des eingesetzten Disulfids und eine Wasserlöslichkeit von weniger als 0,5 g/l aufweist, in einer Reaktionskolonne, wobei anfallende Leichtsieder in Schritt (a) zurückgeführt werden,

   (c) Phasentrennung des erhaltenen Gemisches aus wäßriger Phase, die ausgeschleust wird und schwefelorganischer Phase,

   (d) Aufreinigung der schwefelorganischen Phase, die gegebenenfalls Leichtsieder, das gewünschte organische Disulfid, Polysulfide, Amin und geringe Mengen weiterer Nebenprodukte enthält, durch Destillation, wobei das organische Disulfid entnommen wird, gegebenenfalls anfallende Leichtsieder in Schritt (a) zurückgeführt werden und anfallende Polysulfide und Amin in Schritt (b) zurückgeführt werden, unter Zusatz von Schwefel und gegebenenfalls Amin,

   wobei die Phasentrennung und das Ausschleusen der wäßrigen Phase in Schritt (c) im Anschluß an Schritt (a) oder Schritt (b) erfolgen kann.

2. Verfahren nach Anspruch 1, **dadurch gekennzeichnet, dass** die Aufreinigung in Schritt (d) durch eine Destillation bei vermindertem Druck erfolgt.

3. Verfahren nach Anspruch 1 oder 2 **dadurch gekennzeichnet, dass** der zur Mercaptan-Synthese geeignete Katalysator Kalium-Wolframat auf aktiviertem $Al_2O_3$ enthält.

4. Verfahren nach einem der Ansprüche 1 bis 3, **dadurch gekennzeichnet, dass** das als Lösungsmittel eingesetzte organische Disulfid und das herzustellende organische Disulfid dieselben Verbindungen sind.

5. Verfahren nach einem der Ansprüche 1 bis 4, **dadurch gekennzeichnet, dass** primäre, sekundäre oder tertiäre Amine mit 6 bis 60 Kohlenstoffatomen eingesetzt werden.

6. Verfahren nach einem der Ansprüche 1 bis 5, **dadurch gekennzeichnet, daß** das organische Disulfid Dimethyldisulfid ist.

**Claims**

1. A method for the continuous production of organic disulfides, comprising the following steps

> (a) reaction of alkanols with hydrogen sulfide over a suitable catalyst to give a "crude mercaptan stream" comprising mercaptan, water, hydrogen sulfide and small amounts of other by-products such as organic sulfide and ether,
>
> (b) reaction of the "crude mercaptan stream" with sulfur dissolved in an organic disulfide with catalysis with an amine , which is liquid or solid and has a boiling point above the boiling point of the disulfide used and a solubility in water of less than 0.5 g/l, in a reaction column, where low-boiling components which form are returned to step (a),
>
> (c) phase separation of the resulting mixture comprising aqueous phase, which is removed from the system, and organosulfur phase,
>
> (d) purification of the organosulfur phase, which optionally comprises low-boiling components, the desired organic disulfide, polysulfides, amine and small amounts of other by-products, by distillation where the organic disulfide is taken off, any low-boiling components which form are returned to step (a), and polysulfides which form and amine are returned to step (b), with addition of sulfur and optionally amine,

where the phase separation and the removal from the system of the aqueous phase in step (c) can take place immediately following step (a) or step (b).

2. A method as claimed in claim 1, wherein the purification in step (d) is carried out by distillation at reduced pressure.

3. A method as claimed in claim 1 or 2, wherein the catalyst suitable for mercaptan synthesis comprises potassium tungstate on activated $Al_2O_3$.

4. A method as claimed in any of claims 1 to 3, wherein the organic disulfide used as solvent and the organic disulfide to be prepared are the same compounds.

5. A method as claimed in any of claims 1 to 4, wherein primary, secondary or tertiary amines having from 6 to 60 carbon atoms are used.

6. A method as claimed in any of claims 1 to 5, wherein the organic disulfide is dimethyl disulfide.

**Revendications**

1. Procédé pour la préparation continue de disulfures organiques, comprenant les stades opératoires suivants :

> (a) réaction d'alcanols avec le sulfure d'hydrogène sur un catalyseur approprié, donnant un « courant de mercaptan brut » contenant le mercaptan, de l'eau, du sulfure d'hydrogène et des petites quantités d'autres produits d'accompagnement tels qu'un sulfure organique et un éther,
> (b) réaction du « courant de mercaptan brut » avec du soufre dissous dans un disulfure organique avec catalyse par une amine liquide ou solide ayant un point d'ébullition supérieur à celui du disulfure utilisé et une solubilité dans l'eau inférieure à 0,5 g/l, dans une colonne de réaction, avec recyclage des fractions à bas point d'ébullition au stade (a),
> (c) séparation des phases du mélange obtenu en une phase aqueuse qui est évacuée et une phase organique sulfurée,
> (d) purification de la phase organique sulfurée qui contient le cas échéant des fractions à bas point d'ébullition, le disulfure organique recherché, des polysulfures, une amine et des petites quantités d'autres produits d'accompagnement, par distillation dans laquelle on prélève le disulfure organique, on recycle les fractions volatiles éventuelles au stade opératoire (a) et les polysulfures et l'amine au stade opératoire (b), avec adjonction de soufre et le cas échéant de l'amine,

la séparation des phases et l'évacuation de la phase aqueuse au stade (c) pouvant être réalisées à la suite du stade (a) ou du stade (b).

2. Procédé selon la revendication 1, caractérsisé par le fait que la purification du stade (d) est réalisée par distillation sous vide.

3. Procédé selon la revendication 1 ou la revendication 2, **caractérisé par le fait que** le catalyseur convenant pour la synthèse du mercaptan contient du tungstate de potassium sur alumine activée.

4. Procédé selon l'une des revendications 1 à 3, **caractérisé par le fait que** le disulfure organique utilisé en tant que solvant et le disulfure organique en cours de préparation sont identiques.

5. Procédé selon l'une des revendications 1 à 4, **caractérisé par le fait que** l'on utilise des amines primaires, secondaires ou tertiaires contenant 6 à 60 atomes de carbone.

6. Procédé selon l'une des revendications 1 à 5, **caractérisé par le fait que** le disulfure organique est le disulfure de diméthyle.

Fig. 1

H₂S

ROH

A

RSH', H₂O, H₂S

B

C

H₂O

DADS/NRR'

S₈

NRR'

D

P

EP 1 133 472 B1

8